# FASCICULE DE BREVET EUROPEEN

(11) **EP 3 727 457 B1**
(45) Date de publication et mention de la délivrance du brevet: **20.08.2025**
(21) Numéro de dépôt: 18825725.7
(22) Date de dépôt: 21.12.2018
(51) Int. Cl.: A61K 47/32, A61K 47/34, A61K 31/19, A61K 9/06

(54) **DIFFUSEURS DE COMPOSITION DE SEMIO-CHIMIQUES RÉGULÉS EN FONCTION DE LA TEMPÉRATURE**
VERTEILER FÜR ZUSAMMENSETZUNG AUS SEMIOCHEMIKALIEN, DIE ÜBER DIE TEMPERATUR REGULIERT WERDEN
TEMPERATURE-ADJUSTED SEMIOCHEMICAL COMPOSITION DIFFUSERS

(30) Priorité: 22.12.2017 EP 17209898
(43) Date de publication de la demande: 28.10.2020
(73) Titulaire: Ceva Santé Animale, 33500 Libourne (FR)
(72) Inventeur: GUERRET, Olivier, 46170 Pern (FR); DUFOUR, Samuel, 17620 Saint Agnant (FR)
(74) Mandataire: Cabinet Becker et Associés
(86) Numéro de dépôt international: PCT/EP2018/086781
(87) Numéro de publication internationale: WO 2019/122424

(56) Documents cités:
- EP-A1- 2 926 809
- EP-A1- 3 187 046
- DATABASE CAPLUS [online] CHEMICAL ABSTRACTS SERVICE, COLUMBUS, OHIO, US; 12 May 1984 (1984-05-12), OTSUKA PHARMACEUTICAL CO.: "Controlled release pheromone paste preparation", XP055487284, Database accession no. 1982:610497

## Description

### INTRODUCTION

La présente invention décrit des nouvelles formulations de composés sémio-chimiques pour mammifères dans des diffuseurs à mèche particulièrement efficaces pour réguler la diffusion de plusieurs sémio-chimiques à volatilités différentes sur des périodes de temps long et indépendamment des conditions de température et d'hygrométrie.

La problématique d'apaisement des animaux, qu'ils soient de compagnie ou de rente, est une problématique importante qui a été largement étudiée dans l'art antérieur. En effet, les produits utilisés représentent un poids économique important et il existe pour les propriétaires un besoin de combattre les comportements et les symptômes désagréables des animaux domestiques, en particulier les chiens et/ou les chats et, pour les éleveurs un intérêt économique à apaiser les animaux de production en vue d'augmenter leur productivité et leur bien-être.

Les composés sémio-chimiques sont des substances chimiques pures ou en mélange dont se servent un grand nombre d'espèces animales pour communiquer. Les sémio-chimiques sont répartis dans différentes classes selon les natures de l'émetteur ou du récepteur. Quand l'émetteur et le récepteur sont des individus d'une même espèce, on parle de phéromones. Les mots Phéromones et sémio-chimiques seront utilisés de manière interchangeable dans cette demande.

Il a été montré depuis le début des années 90 que certains mammifères tels que les chats, les chiens, les cochons ou les chevaux étaient sensibles à certaines phéromones spécifiques à chaque espèce. Ces phéromones sont en général constituées d'un mélange d'acides ou d'esters gras à point d'ébullition assez élevés.

On pourra se référer aux brevets WO 201514063A1 ; WO1996023414A1 ; WO1999037297A1, WO2004000336A1 ou GB2345635 pour la description de telles phéromones et pour comprendre leur utilisation.

Le principe d'utilisation de ces phéromones consiste à mimer ce que font les animaux eux-mêmes lorsqu'ils marquent par exemple leur territoire. Par exemple, la demanderesse a développé des pulvérisateurs contenant des solutions alcooliques de phéromones qui peuvent être appliquées sur tout type de support. Une fois l'alcool évaporé les phéromones vont rester sur le support et être détectables par l'animal qui va alors croire être en territoire marqué ce qui l'apaise.

Un autre exemple d'application, consiste à utiliser des diffuseurs d'ambiance à mèche. Ces diffuseurs consistent en une bouteille remplie d'une solution paraffinique de phéromones qui élue le long de la mèche. La partie extérieure de la mèche se situe dans un entrefer chauffé au moyen d'une résistance. L'action de la chaleur sur la mèche dissipe la formulation dans l'habitat. Les phéromones se déposent sur tous les supports de la pièce ce qui a pour effet de rassurer l'animal qui s'y trouve.

Les formulations paraffiniques utilisées à ce jour présentent des concentrations massiques en phéromones de l'ordre de 2 à 5%. Cela veut dire que 95 à 98% de la substance émise dans la pièce est une paraffine.

On comprend alors l'importance du choix de la paraffine qui doit être absolument neutre pour ne pas perturber le comportement de l'animal sans parler d'inconvénients pouvant affecter la perception du produit par le propriétaire de l'animal.

Or, récemment, les paraffines liquides se sont vues imposer la nomenclature de produits toxiques en cas d'aspiration pulmonaire du fait de leur très faible viscosité à bas gradient de cisaillement. Cela implique que tous les produits fabriqués avec lesdites paraffines doivent être étiquetés ce qui affecte la perception des diffuseurs à mèche actuels.

D'autre part, pour être efficaces, les phéromones doivent être diffusées de façon régulière dans le temps, et cette diffusion ne doit pas varier dans le temps en fonction de la température ni de l'hygrométrie dans l'environnement dans lequel elles sont diffusées.

Il est donc important de trouver des solutions de substitution à ces paraffines.

La demanderesse a donc cherché à trouver des solvants qui présentent l'enveloppe de propriétés suivante :
- Solvant non toxique (pour l'homme ou l'environnement)
- Solvant à point d'ébullition > 200°C sous pression atmosphérique
- Solvant sans effet secondaire pour une utilisation domestique (pas de détérioration des supports bois, plastiques ou de leurs revêtements)
- Solvant compatible avec les phéromones de mammifères
- Solvant capable de diffuser selon les mêmes caractéristiques que les paraffines (température de diffuseurs et matériaux des flacons à mèche).

Parmi les solvants présentant au moins les 2 premières caractéristiques, la demanderesse a identifié les polyesters, tels que le Rhodiasolve^{™}, ou les polyglycols, tels que les Dowanol^{™} ou Solvenon^{™}, ou encore tels que le 2-éthylhexylal et le tétraoxaundécane.

Aucun de ces solvants ne répond au cahier des charges entier. Par exemple, les polyesters sont extrêmement polaires et ont tendance à dégrader les plastiques (PVC par exemple) ce qui les rend incompatibles avec une diffusion dans un habitat moderne. Dans le cas des polyéthers, les 4 premiers points du cahier des charges peuvent être remplis mais le dernier est problématique. En effet, lorsqu'on remplace, poids pour poids une paraffine par un polyéther de même point d'ébullition, on observe des cinétiques de diffusion pouvant être jusqu' à 2 fois plus rapides.

Pour résoudre ce dernier problème, la demanderesse a trouvé que des agents viscosifiants polymériques pouvaient être avantageusement utilisés pour régler la cinétique de diffusion sans changer les autres performances selon le cahier des charges.

EP 3 187 046-A1 décrit une composition comprenant une substance sémiochimique volatile et des viscosifiants polymériques copolymères acryliques blocs. Cette composition ne comprend pas de solvant.

### DESCRIPTION DE L'INVENTION

Ainsi la présente invention décrit une formulation de phéromone animale caractérisée en ce qu'elle comprend :
a) Un solvant de type polyéther,
b) Une phéromone animale,
c) Un viscosifiant polymérique,
dans laquelle le viscosifiant polymérique est un copolymère à blocs contenant un bloc acrylique et au moins un bloc méthacrylate de méthyle.
L'invention est telle que présentée dans le jeu de revendications annexé.

De telles formulations de composés sémio-chimiques pour mammifères s'avèrent particulièrement efficaces dans des diffuseurs à mèche pour réguler la diffusion de plusieurs sémio-chimiques à volatilités différentes sur des périodes de temps long et indépendamment des conditions de température et d'hygrométrie.

Selon un mode particulier, la formulation comprend entre 85 et 98% en poids de solvant de type polyéther.

Selon un mode particulier, la formulation comprend de 0,1 à 15% en poids de phéromone animale.

Selon un mode particulier, la formulation comprend entre 0.1 et 10% en poids de viscosifiant polymérique.

Selon un mode plus particulier, il s'agit de formulations de phéromones animales, notamment pour diffuseur à mèche, caractérisées en ce qu'elles contiennent:
de 85 à 99% d'un solvant de type polyéther,
de 1 à 15% de phéromone animale, et
de 0.1 à10% de viscosifiant polymérique.

Selon l'invention, les pourcentages sont exprimés en poids du poids total de la formulation, sauf indication contraire.

De préférence, le solvant selon l'invention est un polyéther de point d'ébullition supérieur à 200°C, et plus préférentiellement, le solvant est le Dowanol^{™} TPnB ou un équivalent chimique d'une autre marque, le tétraoxaundécane ou le 2 éthylhexylal.

Plus particulièrement, les polymères viscosifiants utilisés pour ces formulations sont des polymères ou des copolymères de poids moléculaire moyen supérieur à 10.000 g/mol et inférieur à 100.000 g/mol. Ils sont en particulier faiblement solubles dans les polyéthers.

Par polymère faiblement soluble, la demanderesse entend un polymère dont la solubilité dans le polyéther ne dépasse pas 0.5% à une température ambiante de 20°C mais qui peut être soluble à plus de 5% à une température supérieure à 50°C.

De manière encore plus préférentielle, les viscosifiants polymériques sont des copolymères à bloc contenant un bloc d'acrylate et deux blocs de methacrylate de méthyle. En particulier, les polymères viscosifiants peuvent être encore plus préférentiellement choisis parmi les polymères de la marque Nanostrength^{™} développés par la société Arkema.

Les formulations sont en outre caractérisées en ce qu'elles contiennent entre 0.1% et 10% de phéromone, de préférence entre 0.5% et 5%, en poids.

Les phéromones utilisables dans l'invention sont toutes les phéromones de mammifères. De manière préférentielle, les phéromones sont des phéromones apaisantes de mammifère. Elles sont en particulier les phéromones de mammifères à chaine grasse, et plus particulièrement encore les phéromones des mammifères de type acides ou esters gras choisis dans le groupe constitué par les acides gras possédant de 7 à 20 atomes de carbone, les diacides gras tels que l'acide pimélique ou l'acide azélaique, les alkylglycerols (AKG) ou leurs dérivés et le 2-methyl but-2-enal.

Parmi ces phéromones animales, on peut par exemple citer : les phéromones apaisantes, telles que la fraction F3 (phéromone faciale) pour le chat obtenue selon le brevet EP0724832B1, ou les phéromones mimant les phéromones naturelles de la femelle allaitante, telles que décrite dans WO9937297 (par exemple commercialisée dans les produits tels que Adaptil^{®}, Suilence^{®}), Feliway^{™} Friends contenant la fraction CAP (Cat Appeasing Pheromone), ou les compositions contenant des alkyl glycérols, tels que l'alcool érucique, l'alcool chimilique ou leurs acétals obtenus avec le 2-methyl but-2-énal.

L'homme du métier ajoutera par ailleurs tout stabilisant, tel que notamment des antioxydants ou anti-UV, qui permet en particulier de préserver l'intégrité du mélange pendant toute la durée d'utilisation selon ce qui est déjà largement décrit dans l'art antérieur.

Les antioxydants peuvent être choisis dans le groupe constitué par la vitamine E, l'hydroxyanisole de butyle (BHA), l'hydroxytoluène de butyle (BHT), utilisés seuls ou en mélange. Ces antioxydants protègent la substance sémio-chimique de la dégradation et peuvent être ajoutés dans des quantités, en pourcentage en poids de la composition, allant de 0,1% environ à 3% environ, particulièrement entre 0.5 et 2%.

Les additifs anti-UV peuvent être choisis dans le groupe constitué par le béta-carotène, l'acide p-aminobenzoique, les amines encombrées et les alkoxyamines encombrées utilisés seuls ou en mélange. Ces anti-UV protègent les sémio- chimiques de la dégradation par la lumière et peuvent être ajoutés dans des quantités, en pourcentage en poids de la composition, allant de 0,1% environ à 3% environ, particulièrement entre 0.5 et 2%.

La présente invention concerne également un diffuseur à mèche contenant la formulation selon la présente invention telle que décrite ci-avant.

De telles formulations sont avantageusement utilisées dans des diffuseurs à mèche dans le but d'apaiser des animaux dans des pièces à vivre ou pour l'élevage.

### EXEMPLES

### Exemple 1 : Matériel et méthode de fabrication

Les diffuseurs sont constitués de flacons commerciaux en PET d'une contenance de 50 ml bouchés par un porte mèche en polyamide ou en polypropylène.

Les mèches utilisées sont des mèches commerciales en composite bois achetables chez des fabricants tels que Shangai Prima.

Les polymères additifs sont des copolymères à blocs NanoStrength fabriqués par la société Arkema et qui sont des copolymères à bloc auto-assemblés qui structurent à l'échelle nanométrique. Les références commerciales de ces produits sont : nano strength^{™} M22 et M53.

Le solvant Dowanol^{™} TpnB est acheté auprès de la société Unipex distributeur de ces produits en Europe. Son nom officiel est le 1-[(2-Butoxy-1- methylethoxy)-1-methylethoxy]-2-propanol. Ce solvant ne présente pas de danger pour l'homme ou l'environnement.

Les solubilités à froid et à chaud de ces polymères dans le Dowanol^{™} TpnB sont données dans le tableau suivant :

| | M22 | M53 |
|---|---|---|
| Solubilité à 20°C (% poids) | 0.012% | 0.029% |
| Solubilité à chaud (100°C) (% poids) | >5% | >7% |

Les polymères M22 et M53 répondent donc à la définition de l'invention. Ils ont la particularité de ne pas être solubles à froid (donc de ne pas viscosifier la solution à froid, mais uniquement de viscosifier la solution à chaud).

### Exemple 2 :

Les formulations des exemples suivants sont fabriquées selon le procédé suivant :
On prépare dans un réacteur agité double enveloppe la solution de Dowanol^{™} TpnB contenant la phéromone.

On chauffe ce mélange à 70°C puis on ajoute la quantité souhaitée de polymère M53 additif qu'on maintient sous agitation à 70°C jusqu'à dissolution complète du polymère. La température du mélange est ensuite ramenée à température ambiante.

Les flacons sont ensuite remplis à hauteur de 48g par flacon puis bouchés avec le porte mèche, la mèche et le bouchon à vis.

Un flacon de Feliway^{™} commercial est utilisé comme référence.

Le tableau suivant résume les mélanges qui ont été fabriqués selon l'invention dans ces conditions :

| Exemple | Référence | 2A | 2B | 2C | 2D | 2E | 2F (témoin) |
|---|---|---|---|---|---|---|---|
| Animal | Chat | Chat | Chat | Chat | Chien | Cheval | - |
| Solution (ml) | 48 | 48 | 48 | 48 | 48 | 48 | 48 |
| Phéromone (g) | 0.972 | 1 | 1 | 1 | 1 | 1 | 1 |
| Solvant (nature) | Paraffine | TPnB | TPnB | TPnB | TPnB | TPnB | TPnB |
| Solvant (g) | 45.1 | 43.6 | 43.6 | 43.6 | 43.6 | 43.6 | 43.6 |
| M53 (g) | 0 | 0.22 | 0.044 | 0.088 | 0.22 | 0.22 | 0 |

### Exemple 3 : Comparaison des cinétiques d'évaporation en fonction de la composition en étuves ventilées

De manière à comparer les cinétiques d'évaporation, tous les échantillons sont sur un mur de prise de manière à ce que tous les échantillons s'évaporent dans les mêmes conditions. La température ambiante de la pièce où se déroule l'expérience est comprise entre 21 et 23°C.

L'expérience est répétée 10 fois de manière à avoir une analyse statistique des durées d'évaporation. Les poids de chaque flacon sont ensuite évalués et on en déduit la quantité évaporée depuis le début de l'expérience. Les temps de demi-vie et correspondant à 90% de diffusion sont reportés dans le tableau suivant.

| | Référence | 2A | 2B | 2C | 2F |
|---|---|---|---|---|---|
| T50 (j) | 15 (+/- 1) | 21 (+/- 2) | 12 (+/- 1) | 16 (+/- 1) | 9 (+/-1) |
| T90 (j) | 28 (+/-5) | 39 (+/-8) | 23 (+/-4) | 30 (+/-4) | 18 (+/-3) |

Les résultats illustrent les phénomènes suivants :
La cinétique de la formulation 2F est 1.6 fois plus rapide que celle de la référence. Cela illustre le caractère plus polaire du Dowanol^{™} TpnB comparé au solvant utilisé dans la référence. En effet, la cinétique est principalement liée à l'élution de la formulation dans la mèche composite.

La durée de diffusion dans les formulations 2F,2B, 2C et 2A augmente et cette augmentation est linéairement dépendante du taux de polymère dans les formules.

On constate que le taux de 0.2% de polymère M53 permet de rendre la cinétique de diffusion équivalente à celle de référence.

## Revendications

1. Formulation de phéromone animale, **caractérisée en ce qu'**elle comprend:
a) Un solvant de type polyéther,
b) Une phéromone animale, et
c) Un viscosifiant polymérique,
dans laquelle le viscosifiant polymérique est un copolymère à blocs contenant un bloc acrylique et au moins un bloc méthacrylate de méthyle.

2. Formulation selon la revendication 1, **caractérisée en ce qu'**elle contient entre 85 et 98% en poids de solvant.

3. Formulation selon l'une des revendications 1 et 2, **caractérisée en ce que** le solvant possède un point d'ébullition supérieur à 200°C.

4. Formulation selon l'une des revendications 1 à 3, **caractérisée en ce qu'**elle contient entre 0,1 et 10%, de préférence 0,5 et 5 %, en poids de phéromone animale.

5. Formulation selon l'une des revendications 1 à 4, **caractérisée en ce qu'**elle contient entre 0.1 et 10% en poids de viscosifiant polymérique.

6. Formulation selon la revendication 5, **caractérisée en ce que** le viscosifiant polymérique est un polymère de masse moléculaire comprise entre 10.000 g/mol et 100.000 g/mol.

7. Formulation selon l'une des revendications 1 à 6, **caractérisée en ce que** les phéromones sont des phéromones apaisantes de mammifère.

8. Formulation selon l'une quelconque des revendications 1 à 7, **caractérisée en ce que** le solvant est choisi parmi le 1-[(2-Butoxy-1-methylethoxy)-1-methylethoxy]-2- propanol, le 2-éthylhexylal, le tétraoxaundécane et un de leurs mélanges.

9. Diffuseur à mèche contenant la formulation selon l'une quelconque des revendications 1 à 8.

## Patentansprüche

1. Tierische Pheromonformulierung, **dadurch gekennzeichnet, dass** sie Folgendes umfasst:
a) ein Polyether-Lösungsmittel,
b) ein tierisches Pheromon und
c) einen polymeren Viskositätsbildner,
wobei der polymere Viskositätsbildner ein Blockcopolymer ist, das einen Acrylblock und mindestens einen Methylmethacrylatblock enthält.

2. Formulierung nach Anspruch 1, **dadurch gekennzeichnet, dass** sie zwischen 85 und 98 Gew.-% Lösungsmittel enthält.

3. Formulierung nach einem der Ansprüche 1 und 2, **dadurch gekennzeichnet, dass** das Lösungsmittel einen Siedepunkt von über 200°C besitzt.

4. Formulierung nach einem der Ansprüche 1 bis 3, **dadurch gekennzeichnet, dass** sie zwischen 0,1 und 10 Gew.-%, vorzugsweise 0,5 und 5 Gew.-%, tierisches Pheromon enthält.

5. Formulierung nach einem der Ansprüche 1 bis 4, **dadurch gekennzeichnet, dass** sie zwischen 0,1 und 10 Gew.-% polymeren Viskositätsbildner enthält.

6. Formulierung nach Anspruch 5, **dadurch gekennzeichnet, dass** der polymere Viskositätsbildner ein Polymer mit einer Molekülmasse zwischen 10.000 g/mol und 100.000 g/mol ist.

7. Formulierung nach einem der Ansprüche 1 bis 6, **dadurch gekennzeichnet, dass** die Pheromone beruhigende Säugetierpheromone sind.

8. Formulierung nach einem der Ansprüche 1 bis 7, **dadurch gekennzeichnet, dass** das Lösungsmittel ausgewählt ist aus 1-[(2-Butoxy-1-methylethoxy)-1-methylethoxy]-2-propanol, 2-Ethylhexylal, Tetraoxaundecan und einem ihrer Gemische.

9. Dochtdiffusor, enthaltend die Formulierung nach einem der Ansprüche 1 bis 8.

## Claims

1. Animal pheromone formulation, **characterized in that** it comprises:
a) A polyether solvent,
b) An animal pheromone, and
c) A polymeric viscosifying agent,
wherein the polymeric viscosifying agent is a block copolymer containing an acrylic block and at least one methyl methacrylate block.

2. Formulation according to claim 1, **characterized in that** it contains between 85 and 98% by weight of solvent.

3. Formulation according to one of claims 1 and 2, **characterized in that** the solvent has a boiling point of greater than 200°C.

4. Formulation according to one of claims 1 to 3, **characterized in that** it contains between 0.1 and 10%, preferably 0.5 and 5%, by weight of animal pheromone.

5. Formulation according to one of claims 1 to 4, **characterized in that** it contains between 0.1 and 10% by weight of polymeric viscosifying agent.

6. Formulation according to claim 5, **characterized in that** the polymeric viscosifying agent is a polymer with a molecular mass of between 10,000 g/mol and 100,000 g/mol.

7. Formulation according to one of claims 1 to 6, **characterized in that** the pheromones are mammalian calming pheromones.

8. Formulation according to any one of claims 1 to 7, **characterized in that** the solvent is selected from among 1-[(2-Butoxy-1-methylethoxy)-1- methylethoxy]-2-propanol, 2-ethylhexylal, tetraoxaundecane and a mixture thereof.

9. Wick diffuser containing the formulation according to any one of claims 1 to 8.
